# EUROPÄISCHE PATENTSCHRIFT

(11) **EP 1 284 277 B1**
(45) Veröffentlichungstag und Bekanntmachung des Hinweises auf die Patenterteilung: **20.10.2004**
(21) Anmeldenummer: 01119108.7
(22) Anmeldetag: 08.08.2001
(51) Int. Cl.: C09C 3/06, C09C 1/36, A61K 7/42, C09K 3/14

(54) **Mit Siliziumdioxid umhüllte Metalloxidpartikel**
Silica-coated metal oxide particles
Particule d oxyde de metal recouverte de silice

(43) Veröffentlichungstag der Anmeldung: 19.02.2003
(73) Patentinhaber: Degussa Aktiengesellschaft, 40474 Düsseldorf (DE)
(72) Erfinder: Schumacher, Kai Dr., 65719 Hofheim (DE); Mangold, Helmut Dr., 63517 Rodenbach (DE); Hasenzahl, Steffen Dr., 63477 Maintal (DE); Alff, Harald, 63796 Kahl (DE)

(56) Entgegenhaltungen:
- EP-A- 0 908 494
- EP-A- 0 988 853
- EP-A- 1 078 957
- PATENT ABSTRACTS OF JAPAN vol. 1999, no. 02, 26. Februar 1999 (1999-02-26) & JP 10 297914 A (MITSUBISHI CHEM CORP), 10. November 1998 (1998-11-10)

## Beschreibung

Die Erfindung betrifft mit Siliziumdioxid umhüllte Metalloxidpartikel, ein Verfahren zu ihrer Herstellung und ihre Verwendung.

Metalloxide wie Titandioxid oder Zinkoxid finden weite Verbreitung in Sonnenschutzmitteln. Ihre Wirkung beruht im wesentlichen auf Reflexion, Streuung und Absorption der schädigenden UV-Strahlung und hängt wesentlich von der Primärpartikelgröße der Metalloxide ab.

Nachteilig bei Metalloxiden wie Titandioxid oder Zinkoxid ist ihre photokatalytische Aktivität, durch die Reaktionen ausgelöst werden, die zu Veränderungen von Bestandteilen eines Sonnenschutzmittels führen können.

Es wird versucht die photokatalytische Aktivität dieser Metalloxide zu verringern ohne die UV-abschirmenden Eigenschaften zu reduzieren, indem man sie zum Beispiel mit einer Hülle umgibt.

EP-A-0988 853 beschreibt mit Siliziumdioxid umhüllte Metalloxidpartikel, sowie deren Herstellung und Verwendung als Bestandteil in Sonnenschutzmitteln. Nachteilig hierbei ist, dass diese umhüllten Metalloxidpartikel eine niedrige Oberflächenfunktionalität und einen starken Verwachsungsgrad der Partikel aufweisen. Dadurch wird zum einen das Einarbeiten der Partikel in eine kosmetische Formulierung erschwert, zum anderen ist deren Stabilität bezüglich Sedimentation eingeschränkt. Ferner ist auch nachteilig, dass bei der Herstellung dieser Partikel neben Wasser ein organisches Lösungsmittel zwingend notwendig ist, damit sich eine Hülle ausbilden kann. Diese Lösungsmittel, gemäß EP-A-0 988 853 bevorzugt mit Wasser mischbar, verursachen neben höheren sicherheitstechnischen Vorkehrungen auch einen großen wirtschaftlichen Aufwand um die Lösungsmittel nach Reaktion wieder vom Wasser zu trennen und/oder zu entsorgen. Schliesslich können nach EP-A-0988853 nur Tetraalkoxysilane eingesetzt werden, während zum Beispiel halogensubstituierte Silane explizit ausgeschlossen werden.

Es bestand somit die Aufgabe umhüllte Metalloxidpartikel bereitzustellen, die die Nachteile des Standes der Technik vermeiden. Insbesondere sollen sie sich leicht in kosmetische Formulierungen einarbeiten lassen, in diesen stabil sein und eine geringe photokatalytische Aktivität aufweisen.

Eine weitere Aufgabe ist es ein Verfahren bereitzustellen, das die Nachteile des Standes der Technik vermeidet, insbesondere sollen die Verwendung organischer Lösungsmittel und die Einschränkung der Einsatzstoffe auf Seiten der Siliziumdioxid-Vorläufer auf nur eine Verbindungsklasse vermieden werden.

Gegenstand der Erfindung sind umhüllte Oxidpartikel, bestehend aus einem Kern aus einem Metalloxid und einer den Kern umgebenden Hülle aus Siliziumdioxid, dadurch gekennzeichnet, dass die umhüllten Oxidpartikel eine niedrige Struktur, definiert durch einen fehlenden Endpunkt bei der Dibutylphtalatabsorption, aufweisen.

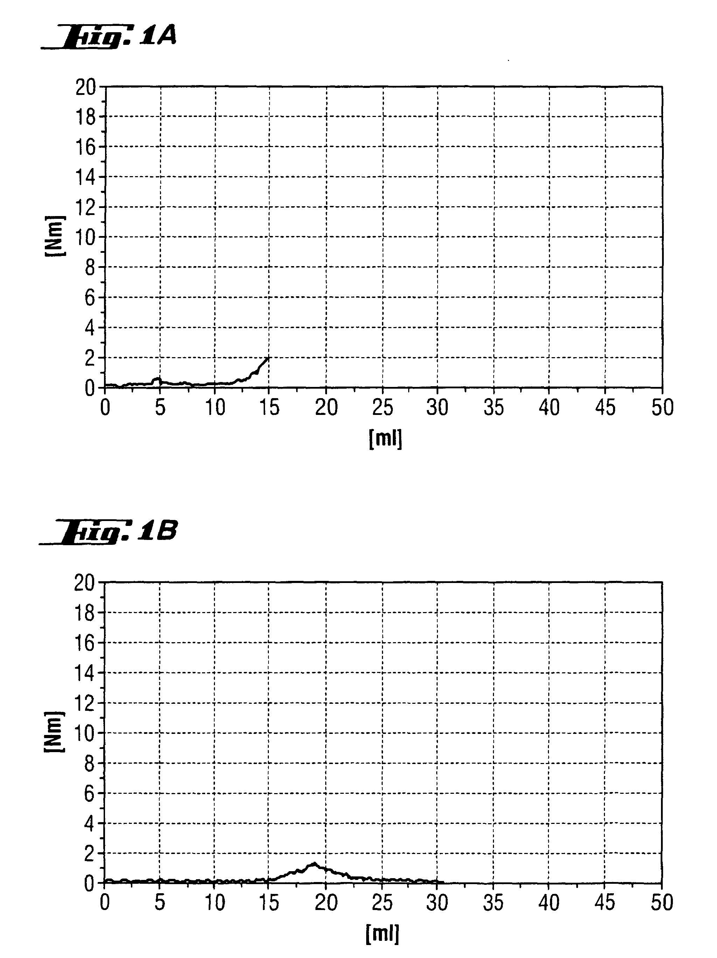

Unter Struktur ist der Verwachsungsgrad der Teilchen zu verstehen, der durch die DBP-Absorption (Dibutylphtalatabsorption) gemessen werden kann. Die niedrige Struktur äußert sich darin, dass kein Endpunkt in der DBP-Absorption zu erkennen ist und bedeutet einen geringen Verwachsungsgrad der Teilchen. Bei der DBP-Absorption wird die Kraftaufnahme, beziehungsweise das Drehmoment (in Nm), der rotierenden Schaufeln des DBP-Meßgerätes bei Zugabe definierter Mengen von DBP, vergleichbar einer Titration, gemessen. Dabei ergibt sich für Metalloxide (zum Beispiel Titandioxid oder Siliziumdioxid, Abb. 1A) ein scharf ausgeprägtes Maximum mit einem anschließenden Abfall bei einer bestimmten Zugabe von DBP. Bei den erfindungsgemäßen Partikeln ist ein Maximum mit anschließendem Abfall nicht zu erkennen, so dass das Gerät keinen Endpunkt ermitteln kann (Abb. 1B).

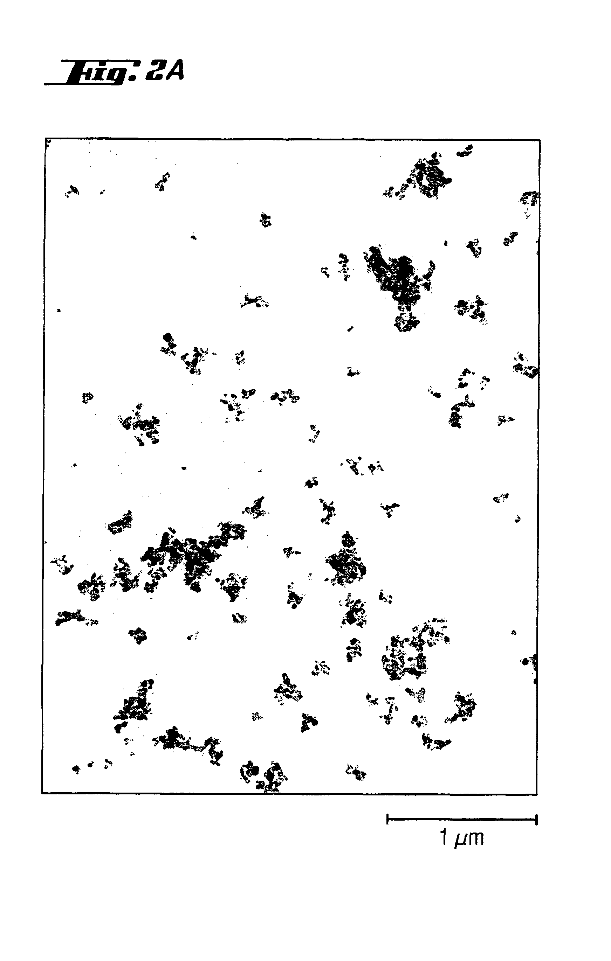

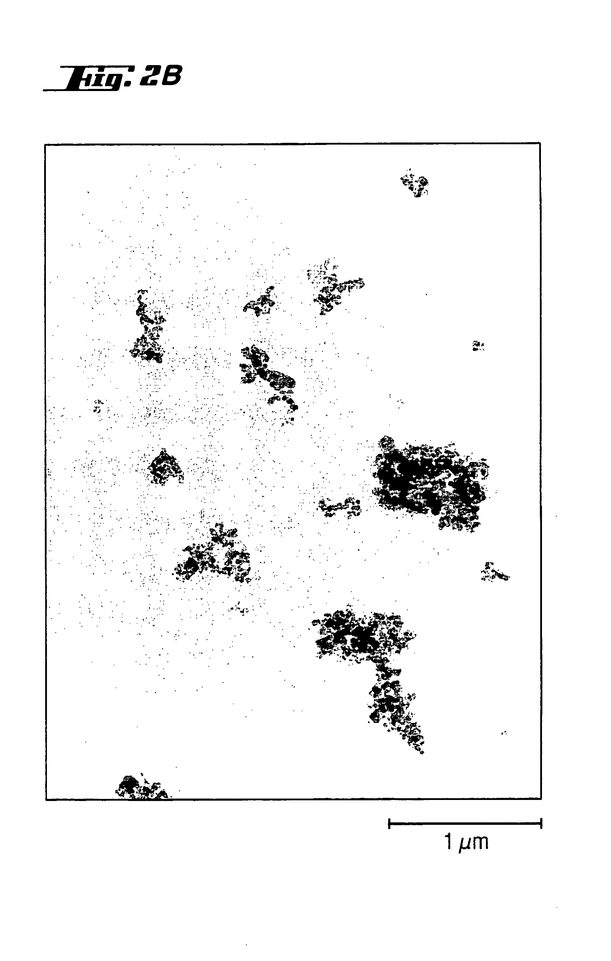

Die niedrige Struktur der erfindungsgemäßen Partikel ist auch den TEM-Aufnahmen zu entnehmen (Abb. 2A). Die nach EP-A-0 988 853 hergestellten Partikel sind deutlich stärker aggregiert (Abb. 2B).

Die erfindungsgemäßen Partikel weisen bevorzugt eine photokatalytische Aktivität von weniger als 0,20·10⁻³ mol kg⁻¹·min⁻¹ auf. Die Aktivität wird bestimmt durch die Oxidation von 2-Propanol zu Aceton durch Bestrahlung mit UV-Licht. Das Ergebnis wird angegeben als Bildungsgeschwindigkeit des Acetons in Form einer Geschwindigkeitskonstanten. Grundlage der Messung ist die von Robert Rudham in "The Chemistry of Physical Sunscreen Materials" (Review derived from a presentation made at the FDA Workshop on the Photochemistry and Photobiology of Sunscreens, Washington, September 19-20, 1996) veröffentlichte Methode. Durch diese niedrige photokatalytische Aktivität können die erfindungsgemäßen Oxidpartikel idealerweise in Sonnenschutzmitteln eingesetzt werden.

Die BET-Oberfläche, bestimmt nach DIN 66131, der erfindungsgemäßen Partikel kann in einem weiten Bereich zwischen 5 und 600 m2/g variiert werden. Gewöhnlich liegt die BET-Oberfläche der erfindungsgemäßen Partikel über der, der zugrunde liegenden Kerne. Bei veränderten Herstellbedingungen kann sie jedoch gegebenenfalls auch kleiner als die der eingesetzten Kerne sein. Bevorzugt jedoch liegt die BET-Oberfläche der erfindungsgemäßen Partikel über der der zugrundeliegenden Kerne.

Die primäre Partikelgröße der umhüllten Oxidpartikel kann zwischen 2 und 100 nm, bevorzugt zwischen 5 und 50 nm, liegen, und die sekundäre Partikelgröße kann zwischen 0,05 und 50 µm, bevorzugt zwischen 0,1 und 1 µm liegen. In diesen Bereichen zeigen die erfindungsgemäßen Partikel bei Verwendung in Sonnenschutzmitteln einen ausreichenden UV-Schutz und ein angenehmes Gefühl auf der Haut nach dem Auftragen.

Die Bestimmung dieser Partikelgrößen erfolgt nach DIN 53206.

Die Schichtdicke der Siliziumdioxidhülle der erfindungsgemäßen Metalloxidpartikel kann zwischen 0,5 und 25 nm variiert werden. In diesem Bereich zeigen die Partikel ausreichend hohe UV-Absorption, Reflexion und Streuung.

Metalloxide können Titandioxid, Zinkoxid, Zirkonoxid, Eisenoxid, Ceroxid und/oder chemische Mischungen dieser Metalloxide untereinander, und/oder chemische Mischungen dieser Metalloxide mit Aluminiumoxid und/oder chemische Mischungen dieser Metalloxide mit Siliziumdioxid sein. Die Herkunft der Metalloxide ist nicht limitiert. So können Metalloxide eingesetzt werden, die aus einem pyrogenen Prozess, einem Sol-Gel, einem Plasma-, einem Fällungsprozess, einem hydrothermalen Verfahren oder durch bergmännische Verfahren oder aus Kombinationen der vorgenannten Verfahren stammen.

Besonders bevorzugte Metalloxide sind die pyrogenen Metalloxide Titandioxid, Zinkoxid, Eisenoxid, Ceroxid, Zirkonoxid und/oder chemische Mischungen dieser Metalloxide untereinander, und/oder chemische Mischungen dieser Metalloxide mit Aluminiumoxid und/oder chemische Mischungen dieser Metalloxide mit Siliziumdioxid.

Unter chemischen Mischungen von pyrogenen Oxiden sind zum Beispiel solche zu verstehen, bei denen eine Komponente über ein Aerosol in den pyrogenen Prozess eingebracht wird, wie in EP-B-0 850 876 beschrieben. Es können auch beide Komponenten gleichzeitig verdampft und in die Mischkammer eines Brenners wie er zur Erzeugung von pyrogenen Oxiden Verwendung findet geleitet werden, wie dies zum Beispiel in EP-A-609 533 für Titan-Silizium- Mischoxid und Titan-Aluminium-Mischoxid oder EP-A-1 048 617 für Silizium-Aluminium-Mischoxid beschrieben ist. Es kann auch ein pyrogenes Metalloxid mit einem weiteren Metalloxid, das in einem nicht pyrogenen Prozeß auf das pyrogene Metalloxid aufgebracht wird, umhüllt oder teilweise umhüllt sein.

Ein weiterer Gegenstand der Erfindung ist ein Verfahren zur Herstellung der erfindungsgemäßen Oxidpartikel. Dazu wird eine in Wasser gelöste Base unter Rühren zu einer Dispersion bestehend aus 1-80 Gew.-% eines Metalloxides, mindestens einer Verbindung der Art XₙSi(OR)₄₋ₙ, wobei das molare Verhältnis XₙSi(OR)₄₋ₙ / Metalloxid je nach Schichtdicke der Siliziumdioxidhülle zwischen 0,1 und 25 liegt, und Wasser gegeben wird, das Reaktionsprodukt abgetrennt, gegebenenfalls gewaschen und getrocknet wird.

Die Abtrennung des Reaktionsproduktes kann erfolgen durch Filtrieren oder Zentrifugieren. Gewaschen werden kann mit Wasser, einem organischen Lösungsmittel oder Mischungen von Wasser mit organischen Lösungsmitteln, wobei Wasser im Sinne der Erfindung bevorzugt ist.

Die erfindungsgemäßen Partikel können nach den, dem Fachmann bekannten, Methoden getrocknet werden. Eine Übersicht über verschiedene Trocknungsverfahren gibt Ullmann's Encyclopedia of Industrial Chemistry, Vol. B2, Unit Operations 1, Seiten 4-2 bis 4-35, 5. Auflage.

Weitere Verfahrensschritte können sich hieran anschließen, wie zum Beispiel Kalzination, Mahlverfahren, Granulationsverfahren, oder eine Dispergierung in geeigneten flüssigen Medien.

Die Temperatur bei der die Reaktion durchgeführt wird ist nicht kritisch, solange das Reaktionsmedium flüssig ist. Bevorzugt wird eine Reaktionstemperatur von 15 bis 30°C.

Die Menge an Base, die benötigt wird kann über einen weiten Bereich von 0,1 bis 30 Gew.-%, bezogen auf das gesamte Reaktionsmedium, variiert werden. Es wurde gefunden, dass eine Basenkonzentration von 1 bis 5 Gew.-% besonders vorteilhaft ist, da bei geringem Baseneinsatz eine rasche Bildung der erfindungsgemäßen Oxidpartikel stattfindet.

Als Base können Ammoniak, Hydroxide wie Natriumhydroxid, Kaliumhydroxid oder Tetraalkylammoniumhydroxid, Carbonate wie Ammoniumcarbonat, Ammoniumhydrogencarbonat, Natriumcarbonat oder Natriumhydrogencarbonat, organische Basen wie Amine, Pyridine, Aniline, Guanidin, AmmoniumSalze von Carbonsäuren wie Ammoniumformiat, Ammoniumacetat, Alkylammonium-Salze von Carbonsäuren wie Monomethylaminformiat, Dimethylaminformiat und deren Mischungen Verwendung finden.

Besonders bevorzugt sind Ammoniak, Ammoniumcarbonat, Ammoniumhydrogencarbonat, Ammoniumformiat, Ammoniumacetat, Natriumcarbonat und Natriumhydrogencarbonat und Mischungen von zwei oder mehr Verbindungen hieraus.

Neben Basen können auch anorganische Säuren, wie zum Beispiel Salzsäure, Schwefelsäure oder Phosphorsäure und organische Säuren wie Ameisen- oder Essigsäure eingesetzt werden um Siliziumdioxid aus der Siliziumdioxidquelle freizusetzen.

Metalloxide können Titandioxid, Zinkoxid, Zirkonoxid, Eisenoxid, Ceroxid, und/oder chemische Mischungen dieser Metalloxide untereinander, und/oder chemische Mischungen dieser Metalloxide mit Aluminiumoxid und/oder chemische Mischungen dieser Metalloxide mit Siliziumdioxid sein. Die Herkunft der Metalloxide ist nicht limitiert. So können Metalloxide eingesetzt werden, die aus einem pyrogenen Prozess, einem Sol-Gel, einem Plasma-, einem Fällungsprozess, einem hydrothermalen Verfahren oder durch bergmännische Verfahren oder aus Kombinationen der vorgenannten Verfahren stammen.

Besonders bevorzugte Metalloxide sind die pyrogenen Metalloxide Titandioxid, Zinkoxid, Eisenoxid, Ceroxid, Zirkonoxid, und/oder chemische Mischungen dieser Metalloxide untereinander, und/oder chemische Mischungen dieser Metalloxide mit Aluminiumoxid und/oder chemische Mischungen dieser Metalloxide mit Siliziumdioxid, wobei wenigstens ein Metalloxid pyrogener Herkunft ist.

Als Verbindungen der Art XₙSi(OR)₄₋ₙ, werden bevorzugt solche eingesetzt bei denen X=Halogen oder H, R=H oder ein linearer oder ein verzweigter Alkylrest mit 1 bis 8 C-Atomen und n = 0-4 mit R ungleich H für n=4, sein kann. Besonders bevorzugt sind Tetraalkoxysilane und/oder deren Oligomere.

Der Vorteil des erfindungsgemäßen Verfahrens ist, dass auf ein organisches Lösungsmittel verzichtet werden kann. Im Gegensatz zu dem in EP-A-0 988 853 beschriebenen Verfahren, bei dem ein organisches Lösungsmittel zwingend zur Ausbildung der Hülle erforderlich ist, werden beim erfindungsgemäßen Verfahren in rascher Reaktion Partikel mit vollständiger Hülle erhalten. Es konnte auch gezeigt werden, dass die so erhaltenen Partikel einheitlich sind, das heißt es wurden ausschließlich die erfindungsgemäßen Partikel nachgewiesen. Partikel, die ausschließlich aus Siliziumdioxid bestehen, entstanden durch das Zusammenwachsen der bei der Hydrolyse der Siliziumdioxidquelle gebildeten feinen SiO₂-Partikel konnten nicht nachgewiesen werden. Offensichtlich besitzen die gemäß der Erfindung eingesetzten Metalloxide eine hohe Affinität zur Siliziumdioxidquelle.

Die erfindungsgemäßen Partikel weisen eine niedrige Struktur auf und sind daher leicht in kosmetische Formulierungen einzuarbeiten, die stabil gegen Sedimentation sind.

Ein weiterer Gegenstand der Erfindung sind Sonnenschutzmittel, die die erfindungsgemäßen Oxidpartikel in einem Anteil von 0,01 und 25 Gew.-% enthalten. Daneben kann das erfindungsgemäße Sonnenschutzmittel in Mischungen mit bekannten anorganischen UV-absorbierenden Pigmenten und/oder chemischen UV-Filtern eingesetzt werden.

Als bekannte UV-absorbierende Pigmente kommen in Betracht Titandioxide, Zinkoxide, Aluminiumoxide, Eisenoxide, Siliziumdioxid, Silicate, Ceroxide, Zirkoniumoxide Bariumsulfat oder Gemische davon in Betracht.

Als chemische UV-Filter kommen alle dem Fachmann bekannten wasser- oder öllöslichen UVA- als auch UV-B-Filter in Frage, von denen exemplarisch, jedoch nicht limitierend Sulfonsäurederivate von Benzophenonen und Benzimidazolen Derivate des Dibenzoylmethans, Benzylidencampher und dessen Derivate, Derivate der Zimtsäure und deren Ester, oder Ester der Salizylsäure genannt seien.

Die erfindungsgemäßen Sonnenschutzmittel können ferner die dem Fachmann bekannten Lösungsmittel wie Wasser, ein- oder mehrwertige Alkohole, kosmetische Öle, Emulgatoren, Stabilisatoren, Konsistenzregler wie Carbomere, Cellulosederivate, Xanthan-Gum, Wachse, Bentone, pyrogene Kieselsäuren und weitere in Kosmetika übliche Stoffe wie Vitamine, Antioxidantien, Konservierungsstoffe, Farbstoffe und Parfums enthalten.

Typischerweise kann das erfindungsgemäße Sonnenschutzmittel als Emulsion (O/W, W/O oder multipel), wässeriges oder wässerig-alkoholisches Gel oder Ölgel vorliegen, und in Form von Lotionen, Cremes, Milchsprays, Mousse, als Stift oder in anderen gebräuchlichen Formen angeboten werden.

Der allgemeine Aufbau von Sonnenschutzmitteln ist darüber hinaus in A. Domsch, "Die kosmetischen Präparate", Verlag für chemische Industrie (Hrsg. H. Ziolkowsky), 4. Aufl., 1992 oder N.J. Lowe und N.A. Shaat, Sunscreens, Development, Evaluation and Regulatory Aspects, Marcel Dekker Inc., 1990 beschrieben.

Ein weiterer Gegenstand der Erfindung ist die Verwendung der erfindungsgemäßen Oxidpartikel als UV-Filter, zur Herstellung von Dispersionen und Verwendung zum chemisch-mechanischen Polieren (CMP-Prozess).

### Beispiele

Die Beispiele 1-6 werden nach dem erfindungsgemäßen Verfahren durchgeführt. Die Vergleichsbeispiele 1-3 werden in Gegenwart eines organischen Lösungsmittels, Ethanol, durchgeführt. Alle Beispiele beinhalten eine Trocknung des Produktes nach Filtration bei Raumtemperatur. Als Base wird eine 29 Gew.-% wässerige Ammoniaklösung eingesetzt.

Die analytischen Daten befinden sich in der den Beispielen nachfolgenden Tabelle.

Die Zusammensetzung von Kern und Hülle wird durch quantitative Röntgenfluoreszenzanalyse, die Schichtdicke der Hülle aus den TEM-Aufnahmen erhalten. Die BET-Oberfläche wird bestimmt nach DIN 66131 und das Porenvolumen der Partikel nach DIN 66134. Die Hydroxylgruppendichte wird bestimmt nach der von J. Mathias und G. Wannemacher in Journal of Colloid and Interface Science 125 (1998) veröffentlichten Methode.

Die Dibutylphthalatabsorption wird gemessen mit einem Gerät RHEOCORD 90 der Fa. Haake, Karlsruhe. Hierzu werden 16 g der beschriebenen Metalloxide auf 0,001 g genau in eine Knetkammer eingefüllt, diese mit einem Deckel verschlossen und Dibutylphthalat über ein Loch im Deckel mit einer vorgegebenen Dosierrate von 0,0667 ml/s eindosiert. Der Kneter wird mit einer Motordrehzahl von 125 Umdrehungen pro Minute betrieben. Nach Erreichen des Drehmomentmaximums wird der Kneter und die DBP-Dosierung automatisch abgeschaltet. Aus der verbrauchten Menge DBP und der eingewogenen Menge der Partikel wird die DBP-Absorption berechnet nach: DBP-Zahl (ml/100 g) = (Verbrauch DBP in ml / Einwaage Partikel in g) x 100.

Abb. 1A zeigt das typische Verhalten von pyrogenen Oxiden mit einem scharf ausgeprägten Maximum mit einem anschließenden Abfall bei einer bestimmten Zugabe von DBP. Abb. 1B zeigt das Verhalten der erfindungsgemäßen Partikel. Hier ist ein Anstieg des Drehmomentes mit anschließendem Abfall bei einer bestimmten Zugabe von DBP nicht zu erkennen. Das DBP-Gerät detektiert keinen Endpunkt.

Abb. 2A zeigt eine TEM-Aufnahme der erfindungsgemäßen Partikel gemäß Beispiel 1, Abb. 2B zeigt bei gleicher Vergrößerung eine TEM-Aufnahme der Partikel, hergestellt gemäß Vergleichsbeispiel 1. Abb. 2A zeigt den deutlichen niedrigeren Verwachsungsgrad der erfindungsgemäßen Partikel.

Bei der Bestimmung der photokatalytischen Aktivität wird die zu messende Probe in 2-Propanol suspendiert und 1h mit UV-Licht bestrahlt. Danach wird die Konzentration an gebildetem Aceton gemessen.

Ca. 250 mg (Genauigkeit 0,1 mg) der aus den Beispielen und Vergleichsbeispielen erhaltenen Partikel werden mit einem Ultra-Turrax-Rührer in 350 ml (275,1g) 2-Propanol suspendiert. Diese Suspension wird mittels einer Pumpe über einen auf 24°C temperierten Kühler in einen zuvor mit Sauerstoff gespülten Photoreaktor aus Glas mit einer Strahlungsquelle gefördert.

Als Strahlungsquelle dient z.B. eine Hg-Mitteldruck-Tauchlampe vom Typ TQ718 (Heraeus) mit einer Leistung von 500 Watt. Ein Schutzrohr aus Borsilikatglas begrenzt die emittierte Strahlung auf Wellenlängen >300nm. Die Strahlungsquelle ist außen von einem mit Wasser durchströmtem Kühlrohr umgeben.

Sauerstoff wird über einen Durchflußmesser in den Reaktor eindosiert. Mit dem Einschalten der Strahlungsquelle wird die Reaktion gestartet. Bei Reaktionsende wird sofort eine eine kleine Menge der Suspension entnommen, filtriert und mittels Gaschromatographie analysiert.

### Beispiel 1:

100 g pyrogenes Titandioxid (P25 von Degussa) werden in 1 l Wasser dispergiert. Zu dieser Lösung werden 100 ml Tetraethoxysilan gegeben. Diese Mischung wird 15 min gerührt, danach erfolgt die Zugabe von 30 ml Ammoniak. Nach 2-4 h Rühren bei 25°C wird das Produkt abfiltriert und getrocknet.

### Beispiel 2:

100 g pyrogenes Titandioxid (P25 von Degussa) werden in 1 l Wasser dispergiert. Zu dieser Lösung werden 200 ml Tetraethoxysilan gegeben. Diese Mischung wird 15 min gerührt, danach erfolgt die Zugabe von 30 ml Ammoniak. Nach 2-4 h Rühren bei 25°C wird das Produkt abfiltriert und getrocknet.

### Beispiel 3:

100 g pyrogenes Titandioxid (P25 von Degussa) werden in 1 l Wasser dispergiert. Zu dieser Lösung werden 100 ml Tetramethoxysilan gegeben. Diese Mischung wird 15 min gerührt, danach erfolgt die Zugabe von 30 ml Ammoniak. Nach 2-4 h Rühren bei 25°C wird das Produkt abfiltriert und getrocknet.

### Beispiel 4:

100 g pyrogenes Titandioxid (P25 von Degussa) werden in 1 l Wasser dispergiert. Zu dieser Lösung werden 1000 ml Tetraethoxysilan gegeben. Diese Mischung wird 15 min gerührt, danach erfolgt die Zugabe von 30 ml Ammoniak. Nach 2-4 h Rühren bei 25°C wird das Produkt abfiltriert und getrocknet.

### Beispiel 5:

100 g eines pyrogenen Titandioxides mit einer BET-Oberfläche von 100m²/g werden in 1 l Wasser dispergiert. Zu dieser Lösung werden 200 ml Tetraethoxysilan gegeben. Diese Mischung wird 15 Minuten gerührt, danach erfolgt die Zugabe von 30 ml Ammoniak. Nach 2-4 h Rühren bei 25°C wird das Produkt abfiltriert und getrocknet.

### Beispiel 6:

100 g mit 0,2% Al₂O₃ dotiertes, pyrogenes Titandioxid (hergestellt nach DE-A-196 50 500) werden in 1 l Wasser dispergiert. Zu dieser Lösung werden 200 ml Tetraethoxysilan gegeben. Diese Mischung wird 15 Minuten gerührt, danach erfolgt die Zugabe von 30 ml Ammoniak. Nach 2-4 h Rühren bei 25°C wird das Produkt abfiltriert und getrocknet.

### Vergleichsbeispiel 1:

100 g pyrogenes Titandioxid (P25 von Degussa) werden in 1,5 l Ethanol und 100 ml Wasser dispergiert. Zu dieser Lösung werden 50 ml Ammoniak gegeben. Anschließend werden zu dieser Mischung 100 ml Tetraethoxysilan in 200 ml Ethanol langsam über einen Zeitraum von 1 h zugetropft. Nach 12 h wird das Produkt abfiltriert und getrocknet.

### Vergleichsbeispiel 2:

400 ml Wasser, 1388 ml Ethanol und 87 ml Ammoniak werden gemischt, anschließend werden darin 105 g Titandioxid dispergiert. Zu dieser Lösung werden über einen Zeitraum von 6 h 193 ml Tetraethoyasilan in 24 ml Wasser und 156 ml Ethanol gegeben. Die Dispersion wird noch 12 h bei 25°C gealtert. Das Produkt wird abfiltriert und getrocknet.

### Vergleichsbeispiel 3:

106 ml Wasser, 480 ml Ethanol und 20 ml Ammoniak werden gemischt, anschließend werden darin 28 g Titandioxid dispergiert. Zu dieser Lösung werden über einen Zeitraum von 2 h 105 ml Tetraethoxysilan in 39,5 ml Wasser und 65,5 ml Ethanol gegeben. Die Dispersion wird noch 12 h gealtert bei 20°C. Das Produkt wird anschließend durch Filtration gewonnen und getrocknet.

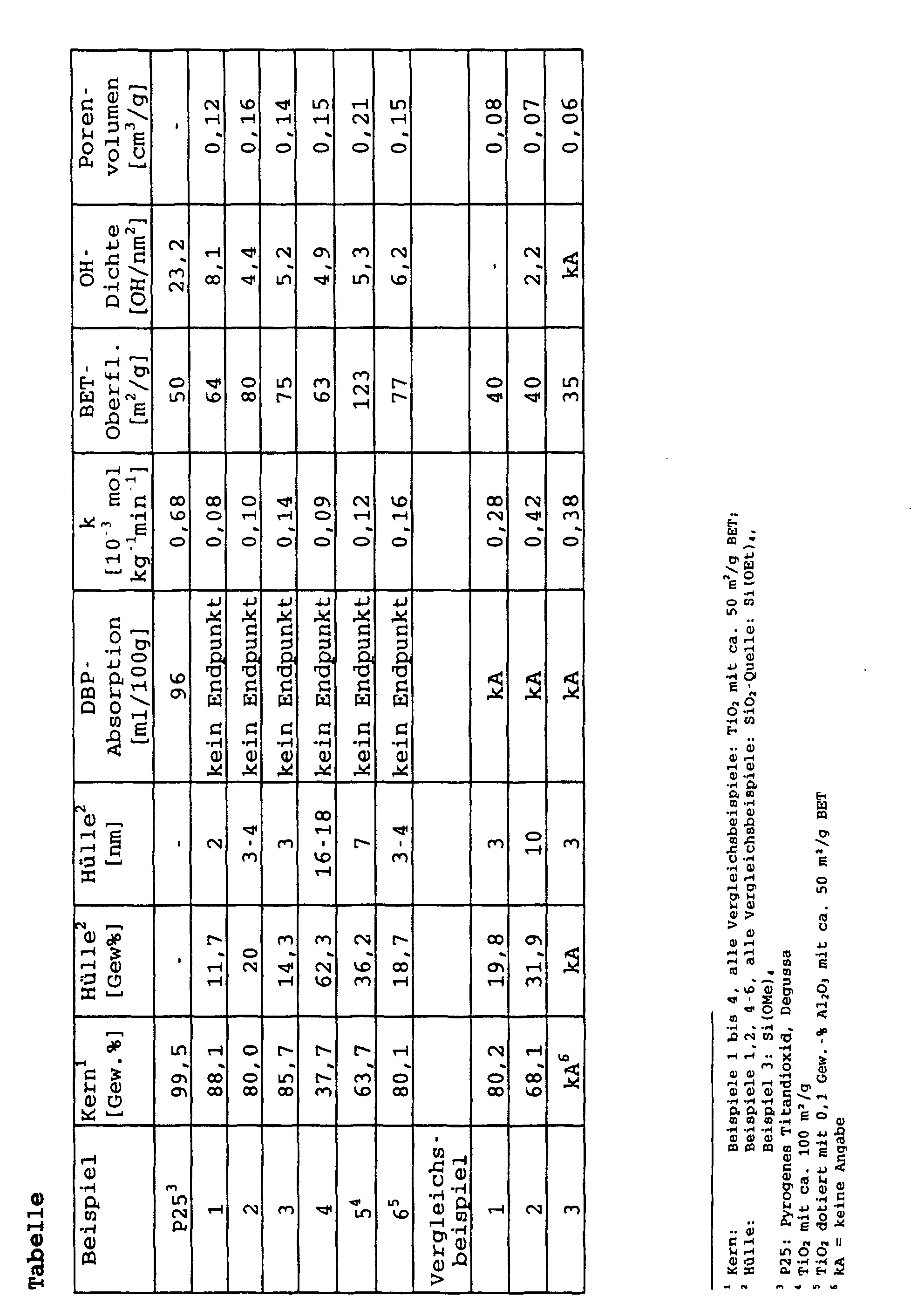

### Sonnenschutzmittel

Mit nachfolgender Rezeptur wurde ein Sonnenschutzmittel mit 4 Gew.-% der erfindungsgemäßen Partikel nach Beispiel 2 hergestellt.

| **Phase** | **Bestandteil** | **Gew.-%** |
|---|---|---|
| A | Isolan GI 34 | 3,0 |
| | Rizinusöl | 1,2 |
| | Tegesoft OP | 10,0 |
| | Tegesoft Liquid | 5,0 |
| | Glycerin 86% | 3,0 |
| B | Paracera W80 | 1,8 |
| | Isohexadecan | 5,0 |
| C | erfindungsgemäßer | 4,0 |
| | Partikel nach Beispiel 2 | |
| D | Magnesiumsulfat | 0,5 |
| | VE-Wasser | 66,5 |

Phase A wird in einem Mischer auf 70°C erwärmt. Nach dem Aufschmelzen auf einer Magnetheizplatte bei 80°C wird Phase B zu Phase A gegeben. Die Phase C wird mit ca. 300 U/min und unter Vakuum in die Ölphase eingerührt. Phase D wird ebenfalls auf 70°C erwärmt und unter Vakuum der Mischung aus A-C zugefügt.

## Patentansprüche

1. Umhüllte Oxidpartrikel bestehend aus einem Kern aus einem Metalloxid und einer den Kern umgebenden Hülle aus Siliziumdioxid, **dadurch gekennzeichnet, dass** - sie einen fehlenden Endpunkt bei der Dibutylphtalatabsorption,
eine photokatalytische Aktivität, bestimmt durch die Oxidation von 2-Propanol zu Aceton durch Bestrahlung mit UV-Licht, kleiner als 0,20·10⁻³ mol · kg⁻¹ · min⁻¹,
eine BET-Oberfläche bestimmt nach DIN 66131, zwischen 5 und 600 m²/g,
eine primäre Partikelgröße zwischen 2 und 100 nm und eine sekundäre Partikelgröße zwischen 0,05 und 50 µm, und
eine Schichtdicke der Siliziumdioxidhülle zwischen 0,5 und 25 nm aufweisen, wobei die Metalloxide Titandioxid, Zinkoxid, Zirkonoxid, Eisenoxid, Ceroxid und/oder chemische Mischungen dieser Metalloxide untereinander, und/oder chemische Mischungen dieser Metalloxide mit Aluminiumoxid und/oder chemische Mischungen dieser Metalloxide mit Siliziumdioxid umfassen.

2. Oxidpartikel nach Anspruch 1, **dadurch gekennzeichnet, dass** die Metalloxide pyrogenes Titandioxid, pyrogenes Zinkoxid, pyrogenes Zirkonoxid, pyrogenes Eisenoxid, pyrogenes Ceroxid und/oder chemische Mischungen dieser Metalloxide untereinander und/oder chemische Mischungen dieser Metalloxide mit Aluminiumoxid und/oder chemische Mischungen dieser Metalloxide Siliziumdioxid, wobei in Mischungen wenigstens ein Metalloxid pyrogener Herkunft ist, umfassen.

3. Verfahren zur Herstellung der Oxidpartikel gemäß den Ansprüchen 1 oder 2, **dadurch gekennzeichnet, dass** eine in Wasser gelöste Base unter Rühren zu einer Dispersion bestehend aus 1-80 Gew.-% eines Metalloxides, mindestens einer Verbindung des Art XₙSi(OR)₄₋ₙ, wobei das molare Verhältnis XₙSi(OR)₄₋ₙ/ Metalloxid je nach Schichtdicke der Siliziumdioxidhülle zwischen 0,1 und 25 liegt, und Wasser gegeben wird, das Reaktionsprodukt abgetrennt, gegebenenfalls gewaschen und getrocknet wird und wobei Verbindungen des Art XₙSi(OR)₄₋ₙ, solche mit X=Halogen, R=H oder ein linearer oder ein verzweigter Alkylrest mit 1 bis 8 C-Atomen und n = 0-4 mit R ungleich H für n=4, sind.

4. Verfahren nach Anspruch 3, **dadurch gekennzeichnet, dass** die Metalloxide Titandioxid, Zinkoxid, Zirkonoxid, Eisenoxid, Ceroxid und/oder chemische Mischungen dieser Metalloxide untereinander und/oder chemische Mischungen dieser Metalloxide mit Aluminiumoxid und/oder chemische Mischungen dieser Metalloxide mit Siliziumdioxid umfassen.

5. Verfahren nach den Ansprüchen 3 oder 4, **dadurch gekennzeichnet, dass** die Metalloxide pyrogenes Titandioxid, pyrogenes Zinkoxid, pyrogenes Zirkonoxid, pyrogenes Eisenoxid, pyrogenes Ceroxid, sowie chemische Mischungen dieser Metalloxide untereinander, und/oder chemische Mischungen dieser Metalloxide mit Aluminiumoxid und/oder chemische Mischungen dieser Metalloxide Siliziumdioxid, wobei in Mischungen wenigstens ein Metalloxid pyrogener Herkunft ist, umfassen.

6. Verwendung der Oxidpartikel nach den Ansprüchen 1 oder 2 als Sonnenschutzmittel, wobei der Anteil an Oxidpartikeln zwischen 0,01 und 25 Gew.-%, bezogen auf die Menge des Sonnenschutzmittels, liegt.

7. Verwendung der Oxidpartikel gemäß den Ansprüchen 1 oder 2 als UV-Filter, zur Herstellung von Dispersionen und in Prozessen zum chemisch-mechanischen Polieren (CMP-Anwendung).

## Claims

1. Coated oxide particles comprising a core of a metal oxide and a coating of silicon dioxide surrounding the core, **characterised in that** the particles exhibit no endpoint during dibutyl phthalate absorption, have a photocatalytic activity, determined by the oxidation of 2-propanol to acetone under irradiation with UV light, of less than 0.20 x 10⁻³ mol x kg⁻¹ x min⁻¹, have a BET surface determined according to DIN 66131 of between 5 and 600 m²/g, have a primary particle size of between 2 and 100 nm and a secondary particle size of between 0.05 and 50 µm, and a layer thickness of the silicon dioxide coating of between 0.5 and 25 nm, wherein the metal oxides comprise titanium dioxide, zinc oxide, zirconium oxide, iron oxide, cerium oxide and/or chemical mixtures of these metal oxides with each other and/or chemical mixtures of these metal oxides with aluminium oxide and/or chemical mixtures of these metal oxides with silicon dioxide.

2. Oxide particles according to claim 1, **characterised in that** the metal oxides comprise pyrogenic titanium dioxide, pyrogenic zinc oxide, pyrogenic zirconium oxide, pyrogenic iron oxide, pyrogenic cerium oxide and/or chemical mixtures of these metal oxides with each other and/or chemical mixtures of these metal oxides with aluminium oxide and/or chemical mixtures of these metal oxides with silicon dioxide, wherein the mixtures contain at least one metal oxide of pyrogenic origin.

3. Process for the production of the oxide particles according to claim 1 or 2, **characterised in that** a base dissolved in water is added under agitation to a dispersion comprising 1-80 wt.% of a metal oxide, at least one compound of the type XₙSi(OR)₄₋ₙ, the molar ratio XₙSi(OR)₄₋ₙ/metal oxide, depending on the layer thickness of the silicon dioxide coating, being between 0.1 and 25, and water, and the reaction product is separated, optionally washed and dried, and wherein compounds of the type XₙSi(OR)₄₋ₙ are those where X=halogen, R=H or a linear or a branched alkyl group with 1 to 8 C atoms and n = 0-4, where R does not equal H when n=4.

4. Process according to claim 3, **characterised in that** the metal oxides comprise titanium dioxide, zinc oxide, zirconium oxide, iron oxide, cerium oxide and/or chemical mixtures of these metal oxides with each other and/or chemical mixtures of these metal oxides with aluminium oxide and/or chemical mixtures of these metal oxides with silicon dioxide.

5. Process according to claim 3 or 4, **characterised in that** the metal oxides comprise pyrogenic titanium dioxide, pyrogenic zinc oxide, pyrogenic zirconium oxide, pyrogenic iron oxide, pyrogenic cerium oxide, as well as chemical mixtures of these metal oxides with each other and/or chemical mixtures of these metal oxides with aluminium oxide and/or chemical mixtures of these metal oxides with silicon dioxide, wherein the mixtures contain at least one metal oxide of pyrogenic origin.

6. Use of the oxide particles according to claim 1 or 2 as sun screening agents, wherein the proportion of oxide particles is between 0.01 and 25 wt.% referred to the amount of the sun screening agent.

7. Use of the oxide particles according to claim 1 or 2 as UV-filters, for the production of dispersions and in processes for chemical-mechanical polishing (CMP application).

## Revendications

1. Particules d'oxyde enrobées, constituées d'un noyau à base d'un oxyde métallique et d'une enveloppe à base de dioxyde de silicium entourant le noyau,
**caractérisées en ce qu'**
elles présentent :
- une absence de point final dans l'absorption de phtalate de dibutyle,
- une activité photocatalytique déterminée par l'oxydation de 2-propanol en acétone par irradiation avec de la lumière UV, inférieure à 0,20.10⁻³ mole.kg⁻¹.min⁻¹.
- une surface BET déterminée selon DIN 66131, comprise entre 5 et 600 m²/g,
- une taille de particule primaire comprise entre 2 et 100 nm et une taille de particule secondaire comprise entre 0,05 et 50 µm, et
- une épaisseur de couche de l'enveloppe de dioxyde de silicium comprise entre 0,5 et 25 nm,
- les oxydes métalliques comprenant le dioxyde de titane, l'oxyde de zinc, l'oxyde de zirconium, l'oxyde de fer, l'oxyde de cérium, et/ou des mélanges chimiques de ces oxydes métalliques entre eux, et/ou des mélanges chimiques de ces oxydes métalliques avec de l'oxyde d'aluminium et/ou des mélanges chimiques de ces oxydes métalliques avec du dioxyde de silicium.

2. Particules d'oxyde selon la revendication 1,
**caractérisées en ce que**
les oxydes métalliques comprennent le dioxyde de titane pyrogéné, l'oxyde de zinc pyrogéné, l'oxyde de zirconium pyrogéné, l'oxyde de fer pyrogéné, l'oxyde de cérium pyrogéné et/ou des mélanges chimiques de ces oxydes métalliques entre eux, et/ou des mélanges chimiques de ces oxydes métalliques avec de l'oxyde d'aluminium et/ou des mélanges chimiques de ces oxydes métalliques avec du dioxyde de silicium, avec dans les mélanges au moins un oxyde métallique d'origine pyrogène.

3. Procédé pour la préparation des oxydes de particules selon la revendication 1 ou 2,
**caractérisé en ce qu'**
une base dissoute dans de l'eau est ajoutée, sous agitation, à une dispersion composée de 1-80 % en poids d'un oxyde métallique, d'au moins un composé du type XₙSI(OR)₄₋ₙ, dont le rapport molaire XₙSI(OR)₄₋ₙ/oxyde métallique est compris entre 0,1 et 25 selon l'épaisseur de couche de l'enveloppe de dioxyde de silicium, et d'eau, on sépare le produit de réaction, éventuellement on le lave, puis on le sèche, les composés du type XₙSI(OR)₄₋ₙ étant ceux dans lesquels X représente un atome d'halogène, R = H ou un radical alkyle linéaire ou ramifié ayant de 1 à 8 atomes de carbone et n = 0-4, R étant différent de H lorsque n = 4.

4. Procédé selon la revendication 3,
**caractérisé en ce que**
les oxydes métalliques comprennent le dioxyde de titane, l'oxyde de zinc, l'oxyde de zirconium, l'oxyde de fer, l'oxyde de cérium et/ou des mélanges chimiques de ces oxydes métalliques entre eux, et/ou des mélanges chimiques de ces oxydes métalliques avec de l'oxyde d'aluminium et/ou des mélanges chimiques de ces oxydes métalliques avec du dioxyde de silicium.

5. Procédé selon la revendication 3 ou 4,
**caractérisé en ce que**
les oxydes métalliques comprennent le dioxyde de titane pyrogéné, l'oxyde de zinc pyrogéné, l'oxyde de zirconium pyrogéné, l'oxyde de fer pyrogéné, l'oxyde de cérium pyrogéné, ainsi que des mélanges chimiques de ces oxydes métalliques entre eux, et/ou des mélanges chimiques de ces oxydes métalliques avec de l'oxyde d'aluminium et/ou des mélanges chimiques de ces oxydes métalliques avec du dioxyde de silicium, avec dans les mélanges au moins un oxyde métallique d'origine pyrogène.

6. Utilisation des particules d'oxyde selon la revendication 1 ou 2, comme produit antisolaire, la proportion de particules d'oxyde étant comprise entre 0,01 et 25 % en poids, par rapport à la quantité du produit antisolaire.

7. Utilisation des particules d'oxyde selon la revendication 1 ou 2, comme filtre UV, pour la préparation de dispersions et dans des processus destinés au polissage chimique-mécanique (utilisation dans le CMP).
